# EUROPEAN PATENT SPECIFICATION

(11) **EP 1 499 711 B1**
(45) Date of publication and mention of the grant of the patent: **29.09.2010**
(21) Application number: 03718573.3
(22) Date of filing: 24.04.2003
(51) Int. Cl.: C12N 5/071

(54) **METHOD FOR PREPARING TISSUE CONSTRUCTS**
VERFAHREN ZUR HERSTELLUNG VON GEWEBEKONSTRUKTEN
METHODE DE PREPARATION DE CONSTRUITS TISSULAIRES

(30) Priority: 24.04.2002 US 374786 P
(43) Date of publication of application: 26.01.2005
(73) Proprietor: Organogenesis, Inc., Canton, MA 02021 (US)
(72) Inventor: AUGER, François A., Sillery, Québec G1S 1A9 (CA); BERGERON, François, Sainte-Foy, Québec G1V 2P5 (CA); GERMAIN, Lucie, Saint-Augustin, Québec G3A 1H8 (CA)
(74) Representative: Modiano, Micaela Nadia
(86) International application number: PCT/CA2003/000617
(87) International publication number: WO 2003/091380

(56) References cited:
- WO-A-00/66036
- US-A- 5 750 329
- US-A- 6 133 030
- L'HEUREUX N ET AL: "A completely biological tissue-engineered human blood vessel" FASEB JOURNAL, vol. 12, 1998, pages 47-57, XP002942845 ISSN: 0892-6638

## Description

### TECHNICAL FIELD

The invention relates to tissue engineering. It also relates to *in vitro* production of bioengineered tissue constructs containing spatially separated populations of living cells with extracellular matrix, and a method of preparation thereof. This method can be applied to the construction of a wide range of tissue constructs requiring spatially distinct domains with specific cell populations, extracellular matrix and mechanical properties.

### BACKGROUND ART

Tissue engineering is the art of reconstructing tissue from isolated cells and extracellular matrix components. One efficient way of reconstructing tissues and organs is to first produce living tissue sheets. These sheets, composed of living cells and extracellular matrix, can be used as a starting material for the craving of tissue-engineered constructs. When mechanically assembled with the appropriate tensile strength and the desired shape, the cells will continue to secrete matrix proteins and adjacent sheets will fuse together to form a solid structure. Moreover, these constructs can be made from autologous cells, thus avoiding the immune rejection of the graft.

Living tissue sheets sufficiently strong to be manipulated can be obtained by several methods. The extracellular matrix in the tissue sheet can be either exogenously added or self-produced by the cells. Cells can be seeded along with exogenous extracellular matrix components to form a tissue sheet (ref Bell). Also, cultured cells can synthesize their own extracellular matrix when incubated in the presence of appropriate reagents. It has long been known that ascorbate stimulates collagen production in cultured cells of mesenchymal origin. When cultured on a plastic surface in the presence of ascorbic acid, fibroblasts and smooth muscle cells will form sheets that eventually detach from the culturing substratum. This auto-assembly method yields tissue with organ-like mechanical properties and devoid of any synthetic material, making it perfectly compatible with the living organism in which it is to be implanted, as shown in L'Heureux et al. (FASEB J. 1998, 12:47-57).

When a tissue reconstructed with layers of different cell types has to be produced, the successive assembly of the different separated sheets increase the production time of the final product. Furthermore, tissue artifacts may appear at the point of fusion between the sheets as prepared in the art.

### SUMMARY OF THE INVENTION

One object of the present invention is to provide a method for assembling living tissue sheets for forming a continuous tissue construct comprising the step of:
a) providing at least two cell populations capable of forming at least two separated living tissue sheets, the cell populations being partially or totally confluent;
b) causing edge contact between the cell populations of step a) for a period of time sufficient for assembling the living tissue sheets into a single continuous tissue construct; and
c) rolling the continuous tissue construct to produce a tubular tissue construct.

The cell populations of step a) may be composed of homologous or heterologous types of cells and are preferably mammalian cells selected from the group consisting of mesenchymal cells, muscle cells, smooth muscle cells or fibroblasts.

Another object of the present invention is to provide single continuous tissue construct composed of at least two living tissue sheets, wherein the living tissue sheets are placed in edge contact for a period of time sufficient for causing assembly of the living tissue sheets into a single continuous tissue construct.

The living tissue -sheets used in the preparation of the single continuous tissue construct can be composed of homologous or heterologous types of cells.

The cell population may comprise at least one type of cells.

The living tissue sheet may comprise at least one cell layer.

The living tissues can be separated by a separator, which can be impermeable or allows selective passage of components contained in a culture medium in which are maintained the cell populations, living tissue sheet, tri-dimensional tissue constructs or tissue grafts.

The contact of cell populations of step b) may be caused by removal of the separator between the at least two living tissue sheets, or by placing the living tissue sheets in contact.

In accordance with the present invention there is provided a tubular tissue construct that is a blood vessel.

According to another aspect of the invention, there is provided a method of forming a continuous living tissue construct comprising allowing edge contact of at least two separated cell populations maintained in culture, each cell population forming a living tissue sheet, for a period of time sufficient for assembling of the cell populations into a single continuous tissue construct.

Another object of the present invention is to provide a method to rapidly produce a living tissue sheet with two or more distinct domains or cell populations, which are perfectly fused or biologically joined together.

Another aspect of the invention is that the cells forming the cell populations, the living tissue sheets or the tri-dimensional tissue constructs, can consist of genetically transformed cells.

In accordance with the invention, there is also provided a method allowing the creation of leak-proof compartments in a tissue culture flask. Each of these compartments can be separately seeded with different cell types and extracellular matrix, resulting in the creation of a composite sheet of tissue that can be folded, stacked, cut, or rolled in order to create the desired tridimensional tissue constructs. A silicone or rubber separator device that is placed in the culture dish before seeding of the cells can be used to achieve a separation between the different cell populations. After the cells have attached to their respective part of the dish surface (1 to 24 hours, depending on the cell type), the separator is removed and the cell populations are allowed to grow and close the gap between them. The composite cell sheet that is formed by this method is completely devoid of any suturing artifacts and its different parts are perfectly fused together. The shape of the separator can be designed to allow a particular motif of cell layers to be produced for further assembly in a tridimensional construct. Many separators may be used to generate multiple cell domains on the sheet.

It will be understood that the cell populations can be embedded into a gel, preferably but not limited to a collagen gel, before being placed in culture for allowing edge contact.

### BRIEF DESCRIPTION OF THE DRAWINGS

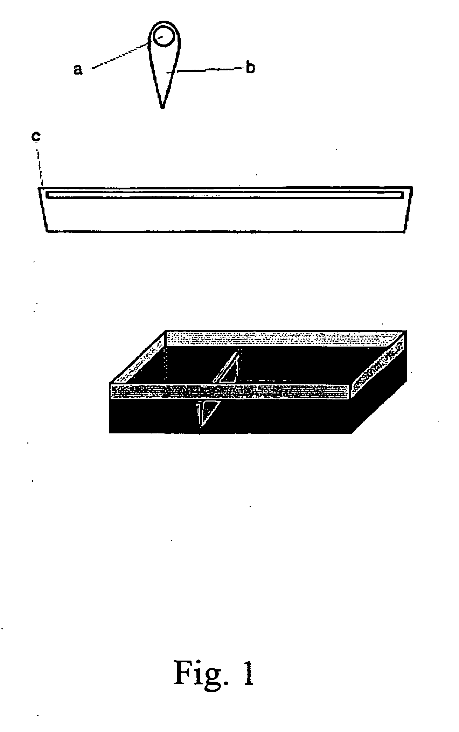

Fig. 1 is a the schematic representation of the method of preparing tissue constructs according to one embodiment of the present invention.

### MODES OF CARRYING OUT THE INVENTION

The present invention will be more fully described hereinafter with reference to the accompanying drawings, in which preferred embodiments of the invention are shown. This invention, may, however, be embodied in many different forms and should not be construed as to be limited to the embodiments set forth herein; rather, these embodiments are provided so that this disclosure will be thorough and complete, and will fully convey the scope of the invention to those skilled in the art.

In many cases, tissue constructs may require that different sheets made of different cell types be assembled together without any suturing technique. This can be achieved by co-culturing the sheets side by side, allowing the cells to organize the matrix between them and to make the sheets fused together. Prior to seeding the cells, a separator, that will create a temporary physical barrier between the flask compartments, is placed in the flask.

The separator can be used with any flask suitable for cell culture. Its design can be made to fit any size and any shape.

This process can be extended to create more than two domains in a sheet by using many separators. The separators can be of different shape and size and can also be shaped to generate a desired pattern.

Another embodiment of the present invention is that the continuous tissue construct can be prepared by placing in edge contact at least two cell populations or tissue domains following separate culture of each population or domain.

The first cell culture chamber can be separated from the second cell culture chamber, by an impermeable or a filtration membrane. A transfer of the cell products between the cell culture chambers may thus be avoided or allowed. The transfer of cell products from one cell culture chamber to the second cell culture chamber can occur continuously or temporarily. The method for producing the culture tissue constructs permits simultaneous culturing of the two cell cultures side by side, for example, in a common culture medium. As the filtration membrane is impermeable to cells, contamination of the cell cultures with cells of the other cell culture is excluded.

After a certain period of time, the separator is removed or the tissue sheets are put in contact to allow edge fusion of cell populations. A matrix can be synthesized at the line of contact of or between the tissue sheets before and/or during fusion of the cell populations. The matrix is generally autoproduced by at least one of the cell populations, and can be composed of different collagen types, fibronectin, or of any other compound produced by the cells that will facilitate fusion of the tissue sheets.

The steps of forming the cellular populations of the tissue construct may be conducted in the presence of growth factors effective to promote proliferation of the cultured cells employed to repopulate the matrix. For example, but without limitation, when fibroblast cells are employed, a growth factor for use herein may be fibroblast growth factor (FGF), most preferably basic fibroblast growth factor. Epithelial growth factors or other cell proliferation and differentiation factors can be used in the preparation of a continuous tissue construct of the present invention.

The fibroblast growth factors (heparin-binding) are a family of mitogens active on mesenchymal cells. FGFs are not detected in free condition in the medium, instead the FGFs are found in the extracellular matrix in association with heparin sulfate, localized in the fibronectin-heparin layer prebound to the transplant tissue matrix. Glycosaminoglycans stabilize FGF activity and are required for FGF binding to cell surface receptors where they stimulate autocrine/paracrine growth.

One embodiment of the invention uses autologous cells in the process described herein. A tissue sample is taken from the recipient prior to transplant or implant surgery. The cells are taken and cultured in accordance with the methods described herein, to produce a continuous tissue construct containing fibroblasts or other cells which are then used to synthesize the allogeneic or xenogeneic tissue construct, in accordance with this process.

The cell source can be selected to match the tissue to be transplanted. For example, if a blood vessel is to be transplanted, cells can be taken from a recipient's healthy blood vessel and used as the source of cells for tissue construct preparation. In this fashion, the healthy tissue construct can be very closely matched to the recipient's diseased tissue.

This aspect is important when the tissue is highly immunogenic, such as when a tissue comprising endothelial cells, and forming, for example, transplantable blood vessels.

Among cell types that can be considered to perform the present invention, but without limitation, , are muscle cells, fibroblasts, and mesenchymal cells. Preferably, muscle cells are smooth muscle cells. Precursor or stem cells can also be used to compose cell populations, living tissue sheets or tri-dimensional tissue constructs.

Alternatively, allogeneic cell lines which are not likely to cause an unacceptable immune response upon implant may be used to prepare the tissue construct. Cells that cause no more than a weak or tolerable immunogenic response may be used to populate the tissue construct to provide a substantially non-immunogenic new or replacement tissue. These cells may be naturally weakly immunogenic or be designed by virtue of recombinant cell technology to be weakly immunogenic.

In one embodiment of the present invention, the continuous tissue construct can be prepared by placing in culture at least two living tissues or cohesive cell populations in a manner to favor edge contact and eventually fusion of the tissue sheets for forming one tissue construct.

According to another embodiment of the present invention, it will be understood that the tissue sheets can be made of several superimposed layers of sheets. This can be defined as being a tri-dimensional tissue construct. Among the tissue substitutes that can be considered, but without limitation, are collagen gels cast with living cells, acellular matrices or synthetic materials. Alternatively, when performing the method of the present invention the tissue sheets can be replaced by biopsies or other tissue substitutes before fusing them into a continuous tissue construct. It will be recognized by someone skilled in the art that the latter, obtained under different configurations or forms, can constitute or be used as a graft or a replacement tissue.

It will be recognized by those skilled in the art to which the present invention pertains that the cells forming the tissue sheets or tri-dimensional tissue constructs can be composed or may comprise genetically transformed cells. Different methods are currently used in the art to genetically transform animal cells. The transformation can be either transient or stable, where the genetic expression DNA vectors are episomic or integrated into the genome of the cells. The cells can also be transformed by using viruses, plasmids, retroviruses, cosmids, or other expression vectors allowing the recombinant production of a protein or peptide of interest in the transformed cells involved in the production of tissue sheets, tissue constructs or continuous tissue constructs as defined herein.

The present invention will be more readily understood by referring to the following examples which are given to illustrate the invention rather than to limit its scope.

### EXAMPLE I

### Apparatus and method for preparing a continuous tissue construct

Fig. 1 depicts the structure of a typical separator. A rigid rod, that can be made of wood, polymer, metal or biological material, may be concealed in an elastomer matrix that is shaped in a way that allows the gap between the cells to be sufficiently small to be covered by the cells once the separator is removed, preferably a blade-like structure with a sharp edge. The assembly has to be made of materials that can be sterilized. At both ends of the rod, a small piece of elastomer functions as a compression fitting that allows the separator to fit tightly in place between the flask walls. When the separator is in place, the edge is in contact with the plastic surface of the culture flask effectively separating the surface in two leak proof compartments. The different cell lines are then seeded in each compartment, and allowed to attach to the surface of the flask before the separator is removed. More than two separators can be used simultaneously to obtain more than two different compartments. This attachment can take 1 to 24 hours, depending on the cell line used (4 hours works well with fibroblasts). The advantage of using a sharp edge on the separator is that only a small gap is created in the cell distribution. As soon as the separator is removed, the cells quickly fill this space, resulting in a continuous cell distribution. The sheet composed of the cells and the extracellular matrix secreted is continuous, allowing the assembly of both cell types into a continuous sheet in one step.

The present invention was applied to the assembly of a tissue-engineered blood vessel. Natural blood vessel walls are formed of two structurally and functionally distinct layers: the inner contractile media, composed of smooth muscle cells (SMC), and the mechanically strong adventice, composed of fibroblasts. In order to reconstruct this structure, one or more sheets of SMCs can be rolled over a solid mandrel to form the contractile media layer and then one or more fibroblast sheets can be rolled over the media layer to produce the adventitia layer.

With the proposed invention, the cell sheets are made as one long continuous sheet composed of two parts: a SMC domain and a fibroblast domain. The size and length of the tissue sheet as well as the cellular domains can be adjusted by selecting appropriate culture flasks. This longer sheet is rolled in the same manner as described above, producing in a single step a complete vessel wall with a dual layer design that mimics the arrangement of the natural vascular wall. The following cell culture methods were used in order to obtain cell sheets: typically, a removable separator was placed in a 500 cm² culture dish in order to produce two distinct compartments. Viable sub-cultured smooth muscle cells and fibroblasts (passages 3-7) were then separately seeded on each side of the separated culture dish with a final seeding density of 10⁴ cells/cm². Cells were fed with 80 ml of culture medium (3:1 mixture of Dulbecco's Modification of Eagle's Medium™ and Ham's F12 Modified Medium™, 10% Fetal Clone II from Hyclone™, 100 U/ml of penicillin G and 25 µg/ml of gentamicin). The culture medium was changed every two days. A freshly prepared solution of ascorbic acid was added every two days to the culture medium at a final concentration of 50 ug/ml. Cells were kept in a humidified atmosphere (92% air and 8% CO₂). Under these conditions the cells will adhere to the culture surface and proliferate until the entire culture surface is covered with cells. The separator has to be in place during the cell adhesion phase and can be removed during the cell proliferation phase or during the sheet formation phase. If the culture conditions are maintained, the cells form a tissue in which multiple cell layers are embedded in an endogenous fibrous material and the sheet formed will eventually detach itself as a whole from the substratum. To roll the living sheet, one edge of the sheet is placed between a tubular support and a thread. The thread squeezes the sheet and holds it in place while the sheet is rolled around the tubular support and finally the sheet is re-secured with a thread to prevent unrolling. Thereafter, the tubular living tissue can be cultured for several weeks, with ascorbic acid, to allow further maturation of the tissue.

## Claims

1. A method for producing a tubular tissue construct comprising (i) allowing edge contact of at least one cell population with the edge of at least another separate cell population maintained in culture, each cell population forming a living tissue sheet, for a period of time sufficient for edge assembling of said cell populations into a single continuous living tissue construct; and (ii) rolling said single continuous living tissue construct to produce a tubular tissue construct.

2. The method of claim 1, wherein said cell populations are partially or totally confluent.

3. The method of claim 1, wherein said cell populations are embedded into a gel before being placed in culture for allowing edge contact.

4. The method of claim 3, wherein said gel is a collagen gel.

5. The method of claim 1, wherein at least one of said cell populations is a single cell layer, a tri-dimensional tissue construct or a tissue graft.

6. The method of claim 1, wherein at least one of said cell populations comprises genetically transformed cells.

7. The method of claim 1, wherein said cell populations are separated by a separator.

8. The method of claim 7, wherein said separator is impermeable or allows selective passage of components contained in a culture medium.

9. The method of claim 1, wherein said tubular tissue construct is a blood vessel.

10. The method of any one of claims 1 to 9, wherein said living tissue sheets are composed of cell populations selected from the group consisting of homologous or heterologous types of cells, mammalian cells, and mesenchymal cells, muscle cells, or fibroblasts.

11. The method of any one of claims 1 to 9, wherein at least one of said cell populations is composed of smooth muscle cells.

12. The method of any one of claims 1 to 9, wherein at least one living tissue sheet comprises at least one type of cells.

13. The method of any one of claims 1 to 9, wherein said living tissue sheet comprises at least one cell layer.

14. The method of any one of claims 1 to 13, wherein said living tissue sheets are placed in edge contact after removal of a separator to form said continuous tissue construct, or by placing cell populations in contact.

15. The method of claim 14, wherein said separator is impermeable or allows selective passage of components contained in a culture medium in which are maintained said living tissue sheets.

## Patentansprüche

1. Ein Verfahren zur Herstellung eines röhrenförmigen Gewebekonstrukts, das folgendes umfasst: (i) Gestatten von Rand-Kontakt von mindestens einer Zellpopulation mit dem Rand von mindestens einer anderen separaten Zellpopulation, in Kultur gehalten, wobei jede Zellpopulation eine lebende Gewebeschicht bildet, für einen Zeitraum, der ausreichend ist zum Rand-Zusammenbau der Zellpopulationen zu einem einzelnen durchgehenden lebenden Gewebekonstrukt und (ii) Einrollen des einzelnen durchgehenden lebenden Gewebekonstrukts, um ein röhrenförmiges Gewebekonstrukt zu bilden.

2. Das Verfahren gemäß Anspruch 1, worin die Zellpopulationen teilweise oder vollständig konfluent sind.

3. Das Verfahren gemäß Anspruch 1, worin die Zellpopulationen in ein Gel eingebettet sind bevor sie in Kultur gegeben werden, um den Rand-Kontakt zu erlauben.

4. Das Verfahren gemäß Anspruch 3, worin das Gel ein Kollagengel ist.

5. Das Verfahren gemäß Anspruch 1, worin mindestens eine der Zellpopulationen eine einzelne Zellschicht, ein dreidimensionales Gewebekonstrukt oder ein Gewebetransplantat ist.

6. Das Verfahren gemäß Anspruch 1, worin mindestens eine der Zellpopulationen genetisch veränderte Zellen umfasst.

7. Das Verfahren gemäß Anspruch 1, worin die Zellpopulationen durch einen Separator getrennt werden.

8. Das Verfahren gemäß Anspruch 7, worin der Separator undurchlässig ist oder den selektiven Durchgang von Komponenten, die in einem Kulturmedium enthalten sind, erlaubt.

9. Das Verfahren gemäß Anspruch 1, worin das röhrenförmige Gewebekonstrukt ein Blutgefäß ist.

10. Das Verfahren gemäß irgendeinem der Ansprüche 1 bis 9, worin die lebenden Gewebeschichten aus Zellpopulationen zusammengesetzt sind gewählt aus der Gruppe bestehend aus homologen oder heterologen Typen von Zellen, Säugetierzellen, und mesenchymalen Zellen, Muskelzellen, oder Fibroblasten.

11. Das Verfahren gemäß irgendeinem der Ansprüche 1 bis 9, worin mindestens eine der Zellpopulationen aus glatten Muskelzellen besteht.

12. Das Verfahren gemäß irgendeinem der Ansprüche 1 bis 9, worin mindestens eine lebende Gewebeschicht mindestens einen Typ von Zellen umfasst.

13. Das Verfahren gemäß irgendeinem der Ansprüche 1 bis 9, worin die lebende Gewebeschicht mindestens eine Zellschicht umfasst.

14. Das Verfahren gemäß irgendeinem der Ansprüche 1 bis 13, worin die lebenden Gewebeschichten nach Entfernung eines Separators in Rand-Kontakt platziert werden, um das durchgehende Gewebekonstrukt zu bilden, oder indem Zellpopulationen in Kontakt miteinander platziert werden.

15. Das Verfahren gemäß Anspruch 14, worin der Separator undurchlässig ist oder den selektiven Durchgang von Komponenten erlaubt, die in einem Kulturmedium enthalten sind, in dem die lebenden Gewebeschichten gehalten werden.

## Revendications

1. Procédé de fabrication d'une confection tissulaire tubulaire, comprenant (i) le placement en contact de bordure d'au moins une population cellulaire avec le bord d'au moins une autre population cellulaire séparée maintenue en culture, chaque population cellulaire formant une feuille de tissu vivant, pour une période de temps suffisante pour l'assemblage en bordure desdites populations cellulaires afin d'obtenir une confection tissulaire vivante continue unique ; et (ii) roulage de ladite confection tissulaire vivante continue unique, pour produire une confection tissulaire tubulaire.

2. Procédé selon la revendication 1, dans lequel lesdites populations cellulaires sont partiellement ou totalement confluentes.

3. Procédé selon la revendication 1, dans lequel lesdites populations cellulaires sont incorporées dans un gel avant d'être placées en culture pour permettre un contact de bordure.

4. Procédé selon la revendication 3, dans lequel ledit gel est un gel collagène.

5. Procédé selon la revendication 1, dans lequel au moins l'une desdites populations cellulaires est une couche cellulaire unique, une confection tissulaire tridimensionnelle, ou une greffe de tissu.

6. Procédé selon la revendication 1, dans lequel au moins l'une desdites populations cellulaires comprend des cellules transformées génétiquement.

7. Procédé selon la revendication 1, dans lequel lesdites populations cellulaires sont séparées par un séparateur.

8. Procédé selon la revendication 7, dans lequel ledit séparateur est imperméable ou permet un passage sélectif de composants contenus dans un milieu de culture.

9. Procédé selon la revendication 1, dans lequel ladite confection tissulaire tubulaire est un vaisseau sanguin.

10. Procédé selon l'une quelconque des revendications 1 à 9, dans lequel lesdites feuilles de tissu vivant composées de populations cellulaires sélectionnées dans le groupe composé de types de cellules homologues ou hétérologues, de cellules de mammifère, et de cellules mésenchymales, de cellules musculaires ou des fibroblastes.

11. Procédé selon l'une quelconque des revendications 1 à 9, dans lequel au moins l'une desdites populations cellulaires est composée de cellules musculaires lisses.

12. Procédé selon l'une quelconque des revendications 1 à 9, dans lequel au moins une feuille de tissu vivant comprend au moins un type de cellules.

13. Procédé selon l'une quelconque des revendications 1 à 9, dans lequel ladite feuille de tissu vivant comprend au moins une couche de cellules.

14. Procédé selon l'une quelconque des revendications 1 à 13, dans lequel lesdites feuilles de tissu vivant sont placées en contact de bordure après enlèvement d'un séparateur, de manière à former ladite confection tissulaire continue, ou par placement de populations cellulaires en contact.

15. Procédé selon la revendication 14, dans lequel ledit séparateur est imperméable ou permet un passage sélectif de composants contenus dans un milieu de culture, dans lequel lesdites feuilles de tissu vivant sont maintenues.
